# EUROPEAN PATENT APPLICATION

(11) **EP 2 572 590 A1**
(43) Date of publication of application: **27.03.2013**
(21) Application number: 11783452.3
(22) Date of filing: 13.05.2011
(51) Int. Cl.: A23L 1/00, A23L 1/015, A23L 1/30, A23L 2/00, A61P 35/00, C12N 1/20, A61K 8/99, A61Q 11/00

(54) **ALDEHYDE REMOVING COMPOSITION**

(30) Priority: 18.05.2010 JP 2010114008
(71) Applicant: Tohoku University, Sendai-shi, Miyagi 980-8577 (JP)
(72) Inventor: NAKAYAMA Toru, Sendai-shi Miyagi 980-8577 (JP); HOSOYA Miho, Sendai-shi Miyagi 980-8577 (JP); YAMAGUCHI Haruhiko, Sendai-shi Miyagi 980-8577 (JP)
(74) Representative: Denison, Christopher Marcus
(86) International application number: PCT/JP2011/061048
(87) International publication number: WO 2011/145518

(57) **Abstract**

An object of the present invention is to provide a composition for removing aldehyde. Specifically, the present invention relates to an aldehyde-removing composition comprising a microorganism belonging to the genus *Gluconobacter* having aldehyde-degrading activity, or extracts thereof or disrupted cells thereof, wherein the microorganism belonging to the genus *Gluconobacter* has higher aldehyde-degrading activity than aldehyde-generating activity.

## Description

### Technical Field

The present invention relates to a composition for removing aldehyde, for example.

### Background Art

An epidemiologic study has revealed that an alcohol drinking habit clearly increases the risk of developing oral cancer, pharyngeal cancer, laryngeal cancer, esophageal cancer, hepatic cancer, rectal cancer/cancer of the colon, or breast cancer (female), and this tendency is true regardless of alcohol type (Non-patent Document 1). Also, an operations committee of the WHO International Agency for Research on Cancer (IARC) has concluded on the basis of the results of various animal experiments that there is sufficient evidence to support the fact that alcohol (ethanol) is carcinogenic to animals.

After alcohol drinking, alcohol is generally converted by alcohol dehydrogenase in the liver to acetaldehyde. Then acetaldehyde is further oxidized by aldehyde dehydrogenase to give acetic acid, and then it is finally degraded into carbon dioxide and water. Acetaldehyde to be generated as an intermediate in the ethanol catabolism process is a causative substance of so-called "bad hangover" symptoms such as flushed face, headache, sleepiness, and nausea/vomiting, and is further certified as a Group I carcinogen (being carcinogenic to humans) by the IARC. Moreover, regarding the carcinogenicity of alcohol, acetaldehyde resulting from alcohol via metabolism is also thought to play a major role.

Furthermore, acetaldehyde causes grassy smell, unusual odor, or degenerative odor in brewed beverages such as beer and rice wine (sake), beer-like alcoholic beverages, and distilled liquors such as shochu (spirits), and it is referred to as a flavor inhibitor (off-flavor). Furthermore, excessive acetaldehyde that is generated from alcohol drinking is also accumulated in saliva to cause odors of liquor mouth ("a dead-wine smell").

A high proportion of the Japanese people have isozyme type2 (ALDH2)-deficient aldehyde dehydrogenase that governs acetaldehyde detoxication (hetero-deficient ALDH2*1/*2: about 40% of the entire population; homo-deficient ALDH2*2/*2: about 10% of the same). People with this genotype exhibit a low acetaldehyde metabolic rate, and thus are of a flusher type whose face becomes significantly reddened by alcohol drinking (Non-patent Document 2). Flushers have a risk of cancer of the upper digestive organ group (oral cavity, pharynx, and esophagus) due to alcohol drinking that is about 50 times higher than that of non-flushers (Non-patent Document 3).

Excessive acetaldehyde is secreted into blood and released by exhaled breath, and also accumulated in saliva. Also, alcohol within the oral cavity is metabolized by oral bacteria into acetaldehyde and then it is accumulated in the upper digestive organ group. In saliva of a flusher (human) with a low acetaldehyde metabolic rate, it is understood that acetaldehyde concentration is significantly increased after alcohol drinking, following which acetaldehyde is circulated/resides within the upper digestive organs for several to more than a dozen hours. It has become revealed that the circulation/retention of acetaldehyde within the upper gastrointestinal tract by alcohol drinking is a major cause of increased risk of developing upper gastrointestinal cancer among flushers. Furthermore, alcoholic beverages, and particularly some distilled liquors originally contain acetaldehyde in minute amounts. There is no clear consensus on whether or not the increased risk of developing cancer due to alcohol is caused by acetaldehyde originally contained in alcoholic beverages or by acetaldehyde newly generated in saliva. In Western countries where the proportion of flushers is low (less than 1% of the entire population), uneven risk distribution of upper gastrointestinal cancer (caused by alcohol drinking) depending on genetic polymorphisms has not become a topic for concern. However, in Japan, where many flushers are present, solving the problem is extremely important for the improvement of people's health and welfare, and is a matter of urgency.

As a means for removing acetaldehyde within the oral cavity after alcohol drinking, the use of L-cysteine (sulfur amino acid), barley green juice, or the like has been examined (Non-patent Document 4). This method has been devised on the basis of the fact that L-cysteine or flavonoid in green juice attaches to acetaldehyde, so as to inactivate acetaldehyde. However, the reaction of forming a complex of L-cysteine or flavonoid in green juice and acetaldehyde is a reversible reaction. This method is problematic in that acetaldehyde may be regenerated. L-cysteine or green juice itself is also a substance having peculiar taste and odor, and the use thereof by ingestion is difficult in practice.

Another possible means for removing acetaldehyde within the oral cavity is a method that involves ingesting a microorganism having aldehyde-degrading activity, so as to degrade acetaldehyde. For example, Patent Document 1 discloses an alcohol-degrading composition containing acetic acid bacteria that degrade alcohol at pH3 or less. Patent Document 1 describes that when acetic acid bacteria exhibiting alcohol-oxidizing enzyme activity even at pH 3 or less are ingested at the time of or before drinking alcohol, alcohol is oxidized to acetic acid via acetaldehyde in the stomach by the acetic acid bacteria, and alcohol is not absorbed in the alimentary canal. However, since such microorganisms actually have activity of oxidizing ethanol in most cases, the method is problematic in that acetaldehyde is unexpectedly generated in an amount higher than that before ingestion from alcohol remaining within the oral cavity as a result of ingestion. It should further be noted that ingredients within foods such as beer or components within saliva significantly enhance the activity of generating acetaldehyde. Therefore, an opposite effect such that ingestion of microorganisms having aldehyde-degrading activity unexpectedly increases the amount of acetaldehyde. Such a problem can be addressed by purifying the activity of generating acetaldehyde from microorganisms or selectively inactivating the activity of generating acetaldehyde through appropriate treatment. However, the use of microorganisms having aldehyde-degrading activity still has practical drawbacks in that noxious substances are generated in an acetaldehyde degrading enzyme reaction, and the addition of a coenzyme is required, for example.

As major acetaldehyde-degrading enzymes of microorganisms, aldehydeoxidase and aldehyde dehydrogenase can be used. Aldehydeoxidase oxidizes aldehyde using molecular oxygen, and then catalyzes a reaction to generate carboxylic acid and hydrogen peroxide. Hydrogen peroxide, which is one of the reaction products, is a noxious substance with carcinogenicity, and thus the generation thereof is not preferred. On the other hand, aldehyde dehydrogenase catalyzes the oxidation of aldehyde using NAD⁺, NADP⁺, or the like as a coenzyme. These coenzymes are extremely expensive. There is a practical need to add a coenzyme in an extremely excessive amount compared with a substrate, or to incorporate an oxidized coenzyme regeneration system, but this is unrealistic because of cost.

### Prior Art Documents

### Patent Document

Patent Document 1: JP Patent Publication (Kokai) No. 9-51778A (1997)

### Non-Patent Documents

Non-patent Document 1: Seitz, H. K., Becker, P., "Alcohol Research & Health", 2007, Vol. 30, pp. 38-47
Non-patent Document 2: Takeshita, T., Morimoto, K., "Environmental Health and Preventive Medicine", 1996, Vol. 1, pp. 1-8
Non-patent Document 3: Akira Yokoyama, "Journal of Clinical and Experimental Medicine (IGAKU NO AYUMI)", 2007, Vol. 222, pp. 643-647
Non-patent Document 4: Salaspuro, V., et al., "Int. J. Cancer", 2002, Vol. 97, pp. 361-364

### Summary of the Invention

### Problems to Be Solved by the Invention

In view of the above circumstances, an object of the present invention is to provide a method for removing acetaldehyde, which is accumulated within the oral cavity after alcohol drinking, from the oral cavity by a safe means. Another object of the present invention is to provide a method comprising degrading acetaldehyde that is a carcinogenic risk factor of upper gastrointestinal cancer among flushers and is accumulated within the oral cavity after alcohol drinking, thus immediately reducing the concentration within the oral cavity to the carcinogen concentration (2.2 ppm) or less. One of the methods is a method for degrading and removing acetaldehyde using food microorganisms, bacteria naturally existing in human bodies such as enteric bacteria, or microorganisms that are present in an environment and have no harmful effects on humans. Objects to be achieved by the method are: i) that no acetaldehyde is generated from alcohol remaining within the oral cavity after alcohol drinking/drinking and eating, or that no special treatment (e.g., purification and heat treatment) is required for prevention of acetaldehyde; ii) that no addition of an oxidized coenzyme (e.g., NAD) is required to accelerate the degradation and removal of acetaldehyde; and iii) that no noxious substance is generated in the process of degrading acetaldehyde, or that no addition of an additive is required for the purpose of degrading noxious substances to be generated in the process of degrading acetaldehyde. In particular, alcohol concentration within the oral cavity after alcohol drinking reaches a level several hundred times to several thousand times greater than the concentration of acetaldehyde. Under such conditions, efficient degradation of acetaldehyde is required. In general, microorganisms capable of degrading acetaldehyde have strong ethanol-oxidizing activity, so that they generate acetaldehyde in the presence of significantly excessive ethanol. Therefore, no microorganism preparation is known by which acetaldehyde accumulated within the oral cavity after alcohol drinking can be removed from the oral cavity.

### Means for Solving the Problem

As a result of intensive studies to achieve the above objects, microorganisms capable of degrading aldehyde such as acetaldehyde, which is a carcinogenic risk factor and causes odor such as a dead-wine smell in the presence of significantly excessive amounts of alcohol, have been searched for, microorganisms meeting the purpose of the present invention have been found from among microorganisms belonging to the genus *Gluconobacter,* and thus the present invention has been completed.

The present invention encompasses the following (1) to (12).

(1) An aldehyde-removing composition comprising a microorganism belonging to the genus *Gluconobacter* having aldehyde-degrading activity, or an extract thereof or disrupted cells thereof, wherein the microorganism belonging to the genus *Gluconobacter* has higher aldehyde-degrading activity than aldehyde-generating activity.

(2) The aldehyde-removing composition according to (1), wherein the microorganism belonging to the genus *Gluconobacter* can degrade aldehyde under conditions where the alcohol concentration is higher than the aldehyde concentration.

(3) The aldehyde-removing composition according to (1), wherein the microorganism belonging to the genus *Gluconobacter* is a microorganism belonging to *Gluconobacter kondonii* or *Gluconobacter kanchanaburiensis.*

(4) The aldehyde-removing composition according to (3), wherein the microorganism belonging to *Gluconobacter kondonii* or *Gluconobacter kanchanaburiensis* is the microorganism specified with NBRC3266 or NBRC103587.

(5) The aldehyde-removing composition according to (1), wherein the aldehyde is acetaldehyde.

(6) The aldehyde-removing composition according to (2), wherein the alcohol is ethanol.

(7) The aldehyde-removing composition according to (1), which is a composition that reduces the risk of developing upper gastrointestinal cancer.

(8) The aldehyde-removing composition according to (7), wherein upper gastrointestinal cancer is selected from the group consisting of oral cancer, pharyngeal cancer, and esophageal cancer.

(9) The aldehyde-removing composition according to (1), which is a composition for deodorization.

(10) The aldehyde-removing composition according to (9), wherein the odor is a dead-wine smell.

(11) The aldehyde-removing composition according to (1), which is used for removing acetaldehyde within the oral cavity after alcohol drinking.

(12) A beverage or a food comprising the aldehyde-removing composition of any one of (1) to (11).

This description includes part or all of the contents as disclosed in the description and/or drawings of Japanese Patent Application No. 2010-114008, which is a priority document of the present application.

### Effects of the Invention

According to the present invention, a composition that is capable of degrading and removing acetaldehyde, which increases the risk of upper gastrointestinal cancer in association with an alcohol drinking habit, and aldehyde, which causes odor, and is capable of fundamentally removing carcinogenic risk factors of the upper gastrointestinal tract and the causes of odor is provided.

### Brief Description of the Drawings

Fig. 1 is a graph showing the generation of acetaldehyde in beverages when cell extracts of the *Gluconobacter oxydans* JCM7642 strain were suspended in various beverages and then incubated at 37°C for 10 minutes.
Fig. 2 is a graph showing the generation of acetaldehyde resulting from reactions between cells belonging to the genus *Gluconobacter* and ethanol.
Fig. 3 is a graph showing the degradation of acetaldehyde by cells of the genus *Gluconobacter* with beer-and-saliva mixed systems.
Fig. 4 is a graph showing the effects of biomass on the removal of acetaldehyde.
Fig. 5 is a graph showing the removal of intermittently added acetaldehyde by cells of the genus *Gluconobacter.*
Fig. 6 is a graph showing the pH dependency of the removal of acetaldehyde by cells of the genus *Gluconobacter.*
Fig. 7 is a graph showing the effects of the ingestion of *Gluconobacter kondonii* cells on the removal of acetaldehyde within the oral cavity after alcohol drinking.

### Embodiments for Carrying Out the Invention

Hereafter, the present invention is described in detail.

The aldehyde-removing composition according to the present invention (hereinafter, referred to as "the composition according to the present invention") contains a microorganism belonging to the genus *Gluconobacter* having aldehyde-degrading activity, or an extract thereof or disrupted cells thereof. Such a microorganism belonging to the genus *Gluconobacter* has higher aldehyde-degrading activity than aldehyde-generating activity, and can degrade aldehyde under conditions where the alcohol concentration is higher than aldehyde concentration. The composition according to the present invention can be used for degrading and removing acetaldehyde, which increases the risk of upper gastrointestinal cancer in association with an alcohol drinking habit, and aldehyde, which causes odor. The composition can also be used as a composition for reducing the risk of developing upper gastrointestinal cancer or a composition for deodorization.

Here, examples of upper gastrointestinal cancer include oral cancer, pharyngeal cancer, and esophageal cancer. According to the WHO IARC classification schema of carcinogens, acetaldehyde associated with alcohol drinking was classified as belonging to Group 1 in 2010, which is confirmed with certainty to be carcinogenic to humans. The carcinogen concentration of acetaldehyde in association with alcohol drinking is estimated to be 2 ppm (50 microM). Therefore, the aldehyde-removing composition according to the present invention, which is capable of immediately lowering the concentration of acetaldehyde within the oral cavity to 2 ppm or less, can be regarded as being capable of reducing the risk of cancer. With the composition according to the present invention, acetaldehyde within the oral cavity after alcohol drinking can be degraded and removed by the ingestion of the composition, and thus acetaldehyde, which increases the risk of upper gastrointestinal cancer in association with an alcohol drinking habit, can be fundamentally removed.

Meanwhile, examples of odor include a dead-wine smell caused by acetaldehyde, body odor such as aging odor caused by nonenal, and a grassy smell caused by hexanal. For example, with the composition according to the present invention, acetaldehyde, which is a substance causing a dead-wine smell within the oral cavity after alcohol drinking, can be degraded and removed by ingestion of the composition, so that the cause of odor can be fundamentally removed. The composition according to the present invention can also be used for removing body odor. Furthermore, the composition according to the present invention can also be used for removing the grassy smell of beverages such as soyinilk or foods.

Examples of an aldehyde compound in the present invention include acetaldehyde, nonenal, and hexanal.

Microorganisms to be used in the present invention have higher aldehyde-degrading activity than aldehyde-(e.g., acetaldehyde) generating activity, and are capable of degrading aldehyde under conditions where the concentration of alcohol (e.g., ethanol) is higher than the concentration of aldehyde (e.g., acetaldehyde). In other words, microorganisms to be used in the present invention are capable of efficiently degrading aldehyde even under conditions where a significantly excessive amount of alcohol such as ethanol (e.g., several hundred times to several thousand times greater in terms of molar ratio) is present compared with aldehyde such as acetaldehyde. An example thereof is a microorganism belonging to the genus *Gluconobacter,* which does not generate acetaldehyde even in the presence of ethanol, is also capable of degrading acetaldehyde without generating acetaldehyde from ethanol in a liquor (e.g., beer)-and-saliva mixed system mimicking a state within the oral cavity after alcohol drinking, and does not generate noxious substances such as hydrogen peroxide in a degrading reaction. Examples of such a microorganism belonging to the genus *Gluconobacter* include microorganisms belonging to species such as *Gluconobacter kondonii* and *Gluconobacter kanchanaburiensis.* Moreover, representative strains that can be used in the present invention include *Gluconobacter kondonii* NBRC3266 and *Gluconobacter kanchanaburiensis* NBRC103587. These strains are available at the Biological Resource Center (NBRC) of the Incorporated Administrative Agency, National Institute of Technology (NITE) (2-5-8 Kazusakamatari, Kisarazu, Chiba, Japan).

Furthermore, microorganisms to be used in the present invention may be bacteria of the genus *Gluconobacter* of either a wild-type strain or a mutant strain, as long as they are capable of efficiently degrading acetaldehyde without generating acetaldehyde in the presence of ethanol, or, capable of degrading acetaldehyde without generating acetaldehyde from ethanol even in a liquor-and-saliva mixed system, and generating no noxious substance such as hydrogen peroxide in a degrading reaction.

Here, the expression "without generating acetaldehyde in the presence of ethanol" means that ethanol is never oxidized at all, or ethanol-oxidizing activity (that is, acetaldehyde-generating activity) is extremely weaker than acetaldehyde-oxidizing activity (that is, acetaldehyde-degrading activity) when the relevant microorganism has weak ethanol-oxidizing activity. For example, ethanol-oxidizing activity is about 1/10, and preferably about 1/50 the acetaldehyde-oxidizing activity.

Furthermore, mutant strains can be obtained by mutagenesis treatment using ethyl methanesulfonate that is a conventionally frequently used mutagenesis agent, treatment with other chemical substances such as nitrosoguanidine, and methyl methanesulfonate, ultraviolet irradiation, or natural mutation without treatment with a mutagenesis agent, for example.

Meanwhile, microorganisms having high acetaldehyde-degrading capacity were searhced for from among many micobial species and many acetic acid bacteria/gluconic acid bacteria, and bacteria of the genus *Pseudomonas* were confirmed to have strong activity. However, these microorganisms exhibited acetaldehyde-generating capacity in the presence of ethanol, and it was revealed that ingredients in foods such as beer or components in saliva further increase such acetaldehyde-generating activity.

Accordingly, microorganisms to be used for the composition according to the present invention can be identified as follows, for example. For example, in a liquor-and-saliva mixed system, a microorganism capable of specifically degrading acetaldehyde without generating acetaldehyde from ethanol is searched for. Strains that satisfy these requirements can be obtained by screening as follows, for example: 1) an appropriate selective medium is coated with a washing for fruit cuticles, a fermented food, human intestinal content, or the like; 2) colonies that have grown are separated; 3) microbial strains capable of efficiently degrading acetaldehyde without generating acetaldehyde in the presence of ethanol are selected, and further, 4) microorganisms capable of degrading acetaldehyde without generating acetaldehyde from ethanol also in a liquor-and-saliva mixed system are obtained; and 5) the presence or the absence of hydrogen peroxide generation in an acetaldehyde-degrading reaction is confirmed by a colorimetric method using peroxidase.

Also, in the present invention, microorganisms capable of degrading other aldehydes (other than acetaldehyde) such as nonenal and hexanal that are odor causative substances can be used. The microorganisms can be identified according to the above screening for microorganisms having acetaldehyde-degrading activity.

Examples of media to be used for growing microorganisms to be used in the present invention include media containing a carbon source (e.g., glucose) and a nitrogen source assimilable by the microorganisms. Examples of a nitrogen source include an organic nitrogen source (e.g., peptone, meat extract, yeast extract, and corn steep liquor), and an inorganic nitrogen source (e.g., ammonium sulfate and ammonium chloride). If desired, media to be used herein may contain a salt comprising cations such as sodium ions, potassium ions, calcium ions, or magnesium ions and anions such as sulphate ions, chlorine ions, or phosphate ions. Furthermore, media can also contain microelements such as vitamins and each of acids. The concentration of a carbon source in a medium ranges from about 0.1% to 10%, for example. Furthermore, the concentration of a nitrogen source in a medium differs depending on type and ranges from about 0.01% to 5%, for example. Moreover, the concentration of inorganic salts in a medium ranges from about 0.001% to 1%, for example. Preferably, acetaldehyde is added to a medium for the purpose of preventing contamination with microorganisms incapable of degrading acetaldehyde. The concentration of acetaldehyde to be added herein can be adequately determined and is preferably 0.1%.

Growth conditions for microorganisms to be used in the present invention are conditions of a temperature ranging from 20°C to 30°C (preferably 25°C to 28°C), at pH 5.0 to 7.0 (preferably pH 6.0 to 6.5) for 1 to 3 days (preferably, 2 days), for example.

The cells of microorganisms in a state of suspension in a culture solution after culture may be used for the composition according to the present invention. Alternatively, the cells of microorganisms recovered or concentrated from a culture solution by a general method such as centrifugation may be used. Recovered or concentrated cells may be immobilized to an appropriate carrier and then used as immobilized cells. Alternatively, recovered or concentrated cells may also be used as powders prepared by a general method such as freeze-drying. The composition according to the present invention can be formulated into dosage forms such as powders, tablets, or capsules by mixing the powders of the cells with various excipients, for example.

Moreover, cell extracts or extracts such as enzyme-extracted fractions or disrupted cells (e.g., containing cell membrane) are prepared by a general method such as ultrasonication or surfactant treatment from microorganisms, and then can also be used for the composition according to the present invention. These extracts and disrupted cells can be used intact, or they can be immobilized to an appropriate carrier and then used as an immobilized enzyme.

To remove acetaldehyde within the oral cavity after alcohol drinking, the composition according to the present invention can be ingested so that dry cells of a microorganism are contained in an amount ranging from 5 mg to 80 mg and preferably ranging from 10 mg to 60 mg, for example.

Meanwhile, the beverage or food according to the present invention contains the compositon according to the present invention. For example, the microorganisms according to the present invention are added to a beverage or food, so that the beverage or food that reduces the risk of upper gastrointestinal cancer or a beverage or food for preventing a dead-wine smell within the oral cavity can be produced.

The beverage or food according to the present invention contains the composition according the present invention such as living cells, dead cells, immmobilized cells, cell extracts, disrupted cells, and acetaldehyde-degrading enzyme preparations purified from microorganisms. With the beverage or food according to the present invention, that is, through ingestion of the composition according to the present invention, acetaldehyde that is present or generated *in vivo* can be removed. To the beverage or food according to the present invention, as long as it has effects of removing acetaldehyde, a sweetener, an acidulant, an aroma chemical, an antioxidant, or the like may be adequately added. Examples of specific product forms include troches, gum, candies, tablets, powders, drinkable preparations, and yogurt. A method for adding an additive or a method for processing into foods, beverages, or the like can be adequately selected and then performed while taking product properties and the like into consideration.

### Examples

Hereafter, the present invention is described in greater detail with reference to the examples, although the technical scope of the present invention is not limited to the examples.

### [Comparative example 1] Acetaldehyde generation in various beverages with the use of Gluconobacter oxydans cell extract

The *Gluconobacter oxydans* JCM7642 strain was pre-cultured using 50 mL of Gluconic acid medium (2% sodium gluconate, 0.3% glucose, 0.3% yeast extract, 0.2% peptone) at 30°C for 3 days. Subsequently, 10 mL of the culture solution after pre-culture was added to 1 L of medium with the same composition, followed by 3 days of culture at 30°C. After culture, cells were recovered by centrifugation (3,000 x g).

1g (wet weight) of cells was suspended in 100 mM potassium phosphate buffer (pH7.0) and then ultrasonicated (intensity 2, 50 cycles, 5 minutes). After recovery of the supernatant by centrifugation, the supernatant was sufficiently dialyzed against the buffer at 4°C. The protein concentration in the dialysis solution was 22 mg/mL.

50 µL of the thus obtained enzyme solution (dialysis solution) was mixed with 550 µL of a beverage (green tea, oolong tea, beer, or whisky), followed by 10 minutes of incubation at 37°C. As a control, an aqueous solution (3 ppm AcH/5% EtOH) containing acetaldehyde (3 ppm) and ethanol (5%) at concentrations that are the same as those originally contained in beer was also tested. 60 µL of 6 M perchloric acid was added to the reaction solution to stop the reaction.

The concentration of acetaldehyde in the reaction solution was measured by headspace gas chromatography. Measurement of the concentration of acetaldehyde by headspace gas chromatography was performed using a Perkin Elmer TurboMatrix 40 headspace autosampler and a SHIMADZU GC-2010 gas chromatograph with an INNOWAX 19091N-233 capillary column (length: 30 m; inner diameter: 0.25 mm; and film: 0.25 µm).

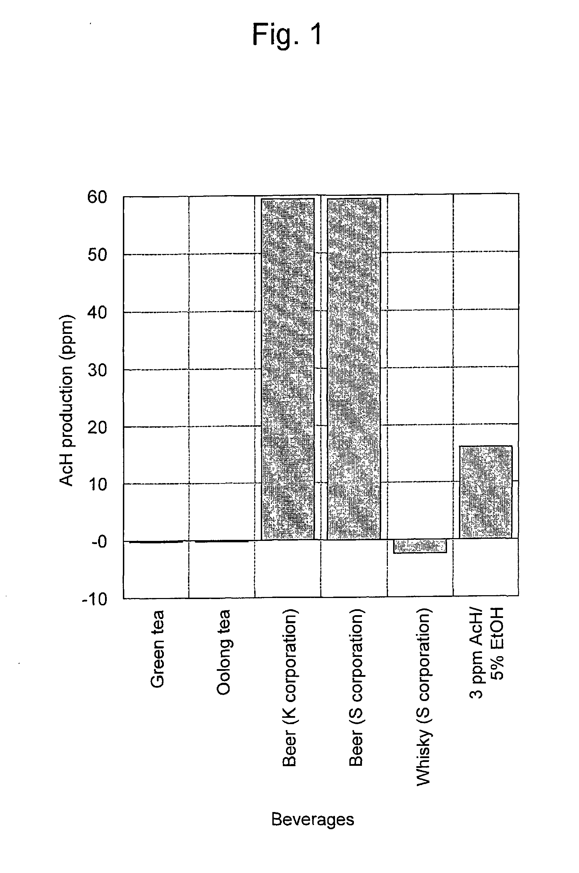

Fig. 1 shows the results of measuring acetaldehyde (AcH). As shown in Fig. 1, particularly significant acetaldehyde generation was observed in the case of beer. The JCM7642 strain is known as a microorganism capable of degrading acetaldehyde. The results indicate that the ingestion of the JCM7642 strain after beer drinking can result in a risk of unexpectedly increasing the concentration of acetaldehyde within the oral cavity because of strong ethanol-oxidizing activity co-existing with acetaldehyde-degrading activity within the cells of the microorganism.

### [Comparative example 2] Reaction of cells of various microorganisms of the genus Gluconobacter with ethanol

5 types of microorganism of the genus *Gluconobacter (Gluconobacter asaii* (*G. asaii*) JCM21279, *Gluconobacter cerinus* (*G.cerinus*) JCM20277, *Gluconobacter sp.* JCM20280, *Gluconobacter thailandicus* (*G. thailandicus*) JCM12310, and *G*. *oxydans* JCM7642) were cultured on Acetobacter agar medium (10% glucose, 1% yeast extract, 3% calcium carbonate, 1.5% agar) at 30°C for 3 days.

After culture, cells of a single inoculating loop were suspended in 50 µL of saline. 50 µL of the cell suspension was added to 50 mM phosphate buffer (550 µL) containing 5% ethanol (final concentration), followed by 10 minutes of incubation at 37°C. 60 µL of 6 M perchloric acid was added to the reaction solution to stop the reaction. Acetaldehyde contained in the reaction solution was measured by the methods described in comparative example 1.

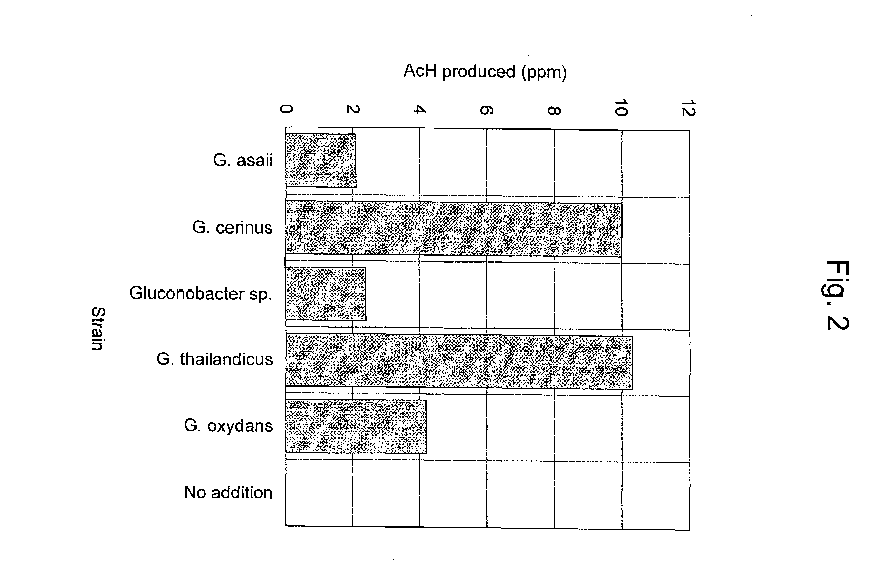

Fig. 2 shows the results of measuring acetaldehyde (AcH). As shown in Fig. 2, all microorganisms generated 2 ppm or more acetaldehyde. Bacteria of the genus *Gluconobacter* are generally known as microorganisms capable of degrading acetaldehyde. The results indicate that the ingestion of these microorganisms after drinking an alcohol beverage can result in a risk of unexpectedly increasing the concentration of acetaldehyde within the oral cavity because of strong ethanol-oxidizing activity co-existing with acetaldehyde-degrading activity within the cells of these microorganisms.

### [Comparative example 3] Reaction of cells of microorganisms of the genus Pseudomonas with ethanol

*Pseudomonas* sp. AL-5 strain known as an acetaldehyde-degrading microorganism was cultured on medium (1.5% agar) at 30°C for 3 days.

After culture, cells of a single inoculating loop were suspended in 50 µL of saline. 50 µL of the cell suspension was added to 50 mM phosphate buffer (550 µL) containing 5% ethanol (final concentration), followed by 10 minutes of incubation at 37°C. 60 µL of 6 M perchloric acid was added to the reaction solution to stop the reaction. Acetaldehyde contained in the reaction solution was measured by the methods described in comparative example 1.

As a result of measurement, cells of the microorganism were found to generate 17.8 ppm acetaldehyde. The results indicate that the ingestion of the microorganism after drinking an alcohol beverage can result in a risk of unexpectedly increasing the concentration of acetaldehyde within the oral cavity because of strong ethanol-oxidizing activity co-existing with acetaldehyde-degrading activity within the cells of the microorganism.

### [Example 1] Reaction of cells of microorganisms of the genus Gluconobacter with beer

*Gluconobacter frateurii* (*G. frateurii*) JCM20278, G. *kondonii* NBRC3266, and *G*. *kanchanaburiensis* NBRC103587 were cultured in a manner similar to that described in comparative example 1. Subsequently, these 3 types of microorganism and 5 types of microorganism of the genus *Gluconobacter* described in comparative example 2 were each suspended in 500 µL of MilliQ water so that each optical turbidity at 600 nm was about 0.9.

250 µL of 50 mM phosphate buffer (pH 7.0) and 300 µl of beer were mixed, and then 50 µL of the cell suspension was added, followed by 10 minutes of incubation at 37°C. 60 µL of 6 M perchloric acid was added to the reaction solution, so as to stop the reaction. Acetaldehyde contained in each reaction solution was measured by the methods described in comparative example 1. As a result, in systems to which *G*. *asaii, G. cerinus, G. frateurii, Gluconobacter sp., G. thailandicus,* and *G*. *oxydans* had been added, respectively, acetaldehyde generation was observed at 22 ppm, 8 ppm, 32 ppm, 9 ppm, 43 ppm, and 17 ppm, respectively. The results indicate that regarding the aforementioned 6 types of Gluconobacter, there is a risk of unexpectedly increasing the concentration of acetaldehyde within the oral cavity after alcohol drinking since ethanol-oxidizing activity co-existing with acetaldehyde-degrading activity within cells of the microorganisms is highly activated by unknown ingredients in beer (which may be vitamins or derivatives thereof). Meanwhile, regarding *G. kondonii* NBRC3266 and *G*. *kanchanaburiensis* NBRC103587, acetaldehyde-degrading activity was significantly higher than acetaldehyde-generating activity even in the presence of such unknown ingredients, and acetaldehyde (3.5 ppm) originally contained in beer was degraded (residual acetaldehyde concentration: 0.2 ppm). This was further confirmed with good reproducibility in the following Example 2.

### [Example 2] Removal of acetaldehyde in saliva using freeze-dried Gluconobacter kondonii cells

*G. kondonii* NBRC3266 was cultured by the method described in Example 1, and then the thus obtained cells were subjected to freeze-drying. About 30 mg of freeze-dried cells was suspended in 500 µL of MilliQ water.

50 µL of a cell suspension was added to a beer-and-saliva mixed solution (volume ratio: 1:1, 550 µL) containing acetaldehyde (initial concentrations: 2.5 ppm and 9.5 ppm), followed by 10 minutes of incubation at 37°C. Also, for comparison, 50 µL of the same cell suspension was added to 550 µL of 0.1% ethanol, followed by 10 minutes of incubation at 37°C. 60 µL of 6 M perchloric acid was added to each reaction solution to stop the reaction. Furthermore, acetaldehyde contained in the reaction solution was measured by the method described in comparative example 1.

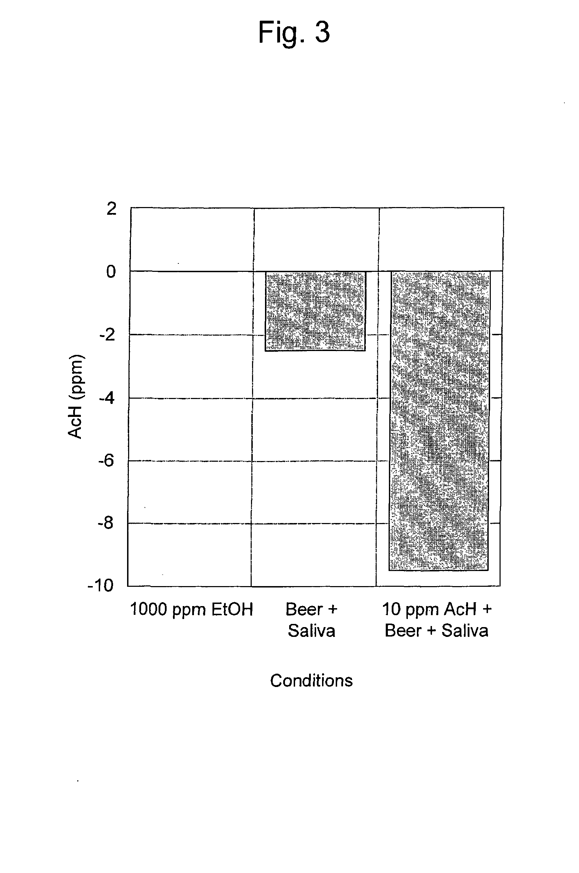

Fig. 3 shows the results of measuring acetaldehyde (AcH). As shown in Fig. 3, no acetaldehyde generation was observed even in the presence of 0.1% ethanol or in the beer-and-saliva mixed system, and acetaldehyde in the beer-and-saliva mixed system was efficiently degraded by freeze-dried cells.

Meanwhile, 0.05 mL of 20 mM 2,4-dichlorophenol, 0.05 mL of 20 mM 4-aminoantipyrine, 0.2 mL of water, and 0.1mL of 10 mg/mL horseradish peroxidase were added to each reaction solution (0.6 mL) obtained in a manner similar to the above except for setting the reaction time at 0, 2, 5, and 10 minutes. Incubation was performed at room temperature for 20 minutes until absorbance of the reaction solution at 505 nm reached a predetermined level. The concentration of hydrogen peroxide contained in each reaction solution was estimated from the increase in absorbance at 505 nm. As a result, the concentrations of hydrogen peroxide in all the reaction solutions were found to be at or below the detection limit of 0.5 µM (0.02 ppm) of the method.

### [Example 3] Time course and specific activity of removal of acetaldehyde

*G. kondonii* NBRC3266 was cultured by the method described in Example 1, and then the thus obtained cells were subjected to freeze-drying. 10 mg of freeze-dried cells was suspended in 500 µL of MilliQ water.

1:1 mixtures of beer and 50 mM potassium phosphate buffer (pH7.0) were prepared, and then acetaldehyde was added to 7.8 ppm. 50 µL each of cell suspensions with different cell concentrations was added to 550 µL (5 solutions in total) of each mixture, followed by incubation at 37°C. At 0, 1, 3, 5, and 10 minutes after the reaction, 60 µL of 6M perchloric acid was added to each reaction solution to stop the reaction. Acetaldehyde contained in these reaction solutions was measured by the method described in comparative example 1.

As a result of measuring acetaldehyde, it was demonstrated that acetaldehyde in the reaction systems was almost completely degraded within 1 minute in the case of this biomass. Based on the results, the acetaldehyde degradation rate per mg of freeze-dried *G*. *kondonii* NBRC3266 cells under the conditions (37°C) was estimated to be 3.9 µg•min⁻¹•(mg dry cells)⁻¹.

### [Example 4] Effects of biomass on removal of acetaldehyde

*G. kondonii* NBRC3266 was cultured by the method described in Example 1, and then the thus obtained cells were subjected to freeze-drying. 10 mg of freeze-dried cells was suspended in 500 µL of MilliQ water. The suspensions were further diluted with MillQ water, so that suspensions with different cell concentrations were prepared.

25 mM potassium phosphate buffer (pH7.0) containing 2.5% ethanol was prepared and then acetaldehyde was dissolved in the solution. 50 µL each of cell suspensions adjusted to have different cell concentrations was added to 550 µL of the mixture, followed by 1 minute or 5 minutes of reaction at 37°C. The initial concentrations of acetaldehyde were 10.1 ppm and 11.1 ppm, respectively, in 1 minute of reaction and 5 minutes of reaction. 60 µL of 6 M perchloric acid was added to the reaction solution to stop the reaction. Acetaldehyde contained in the reaction solutions was measured by the method described in comparative example 1.

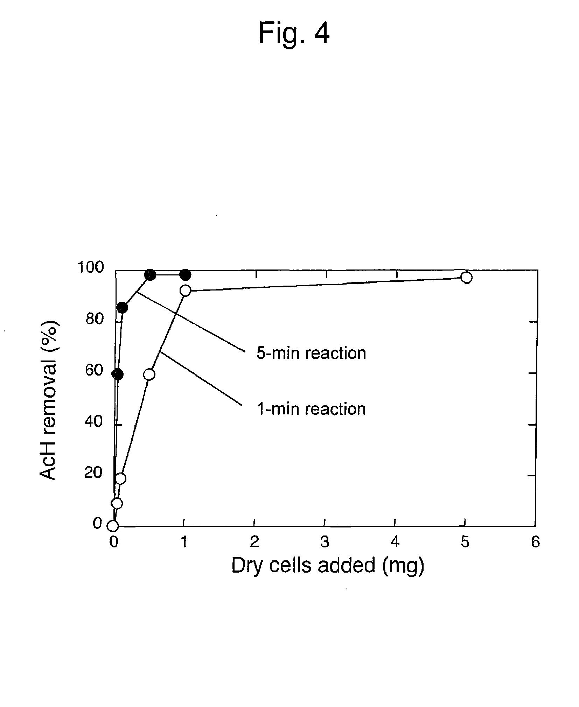

Fig. 4 shows the results of measuring acetaldehyde (AcH). In Fig. 4, white circles indicate 1 minute of reaction and black circles indicate 5 minutes of reaction.

As understood from Fig. 4, 90% or more acetaldehyde can be removed by the use of 0.5 mg of cells in the case of 5 minutes of reaction and 1.0 mg of cells in the case of 1 minute of reaction. Furthermore, the rate of removing acetaldehyde by cells was estimated from the example to be about 3.9 µg • min⁻¹ • (mg dry cells)⁻¹.

### [Example 5] Removal of intermittently added acetaldehyde

*G. kondonii* NBRC3266 was cultured by the method described in Example 1, and then the thus obtained cells were subjected to freeze-drying. 30 mg of freeze-dried cells was suspended in 500 µL of MillQ water.

25 mM potassium phosphate buffer (pH7.0) containing 1.5% ethanol was prepared, acetaldehyde was dissolved to about 10 ppm, and then the solution was maintained at 37°C. The cell suspension (200 µL) was added to the reaction system (1.80 mL) and then incubation was performed at 37°C for 3 minutes. 200 µL of the solution was sampled from the reaction system, and then mixed with 20 µL of 6 M perchloric acid to stop the reaction.

Also, 200 µL of 100 ppm aqueous acetaldehyde solution was added to the remaining reaction solution (1.80 ml), and then incubation was further continued at 37°C for 3 minutes. 200 µL of the solution was sampled from the reaction system, and then mixed with 20 µL of 6M perchloric acid, so as to stop the reaction. This procedure was repeated 3 times (in total) every 3 minutes.

Acetaldehyde contained in each reaction solution for which the reaction had been stopped was measured by the method described in comparative example 1. In addition, a control experiment was conducted by the same procedure except for replacing the addition of an aqueous acetaldehyde solution (200 µL) after sampling of the reaction solution with the addition of water (200 µL).

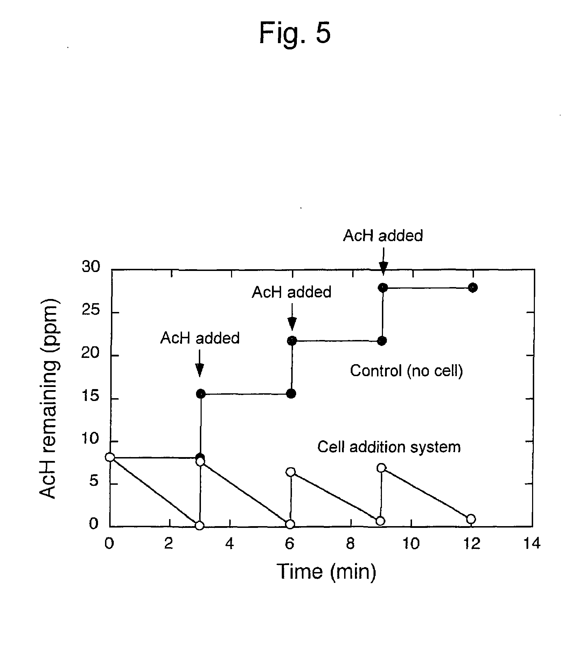

Fig. 5 shows the results of plotting the concentrations of acetaldehyde (AcH) contained in the reaction solutions at each reaction time. Whereas in the control experiment, the concentration was increased cumulatively every time acetaldehyde was added, the concentration of acetaldehyde was maintained between 0.1 ppm and 0.8 ppm in the system to which cells had been added.

### [Example 6] Effects of ethanol concentrations on removal of acetaldehyde

*G. kondonii* NBRC3266 was culutred by the method described in Example 1, the thus obtained cells were subjected to freeze-drying. 10 mg of freeze-dried cells was suspended in 500 µL of MilliQ water.

25 mM potassium phosphate buffer (pH 7.0) solutions containing different concentrations of ethanol were prepared, and then acetaldehyde (6 ppm to 8 ppm (final concentration)) was dissolved. The cell suspension (50 µL) was added to 550 µL each of the mixtures, followed by 5 minutes of reaction at 37°C. 60 µL of 6 M perchloric acid was added to each reaction solution to stop the reaction. Acetaldehyde contained in the reaction solutions was measured by the method described in comparative example 1.

As a result, the cells efficiently degraded acetaldehyde almost completely in the reaction systems containing ethanol with ethanol concentrations of 10% or less (the amount of remaining acetaldehyde: 0.07 ppm to 0.19 ppm). However, in the reaction systems with ethanol concentrations of 20% or higher, AcH-degrading capacity was decreased and the amounts of acetaldehyde remaining in the systems containing 20%, 35%, and 50% ethanol were 3.4 ppm, 5.5 ppm, and 5.2 ppm, respectively.

### [Example 7] Effects of pH on removal of acetaldehyde

*G. kondonii* NBRC3266 was cultured by the method described in Example 1, and then the thus obtained cells were subjected to freeze-drying. 10 mg of freeze-dried cells was suspended in 500 µL of MilliQ water.

Buffer solutions with various pHs containing 2.5% ethanol were prepared, and then acetaldehyde (10.5±0.5 ppm (final concentration)) was dissolved. The cell suspension (50 µL) was added to 550 µL each of the mixtures, followed by 5 minutes of reaction at 37°C. 60 µL of 6 M perchloric acid was added to each reaction solution to stop the reaction. Acetaldehyde contained in the reaction solutions was measured by the method described in comparative example 1.

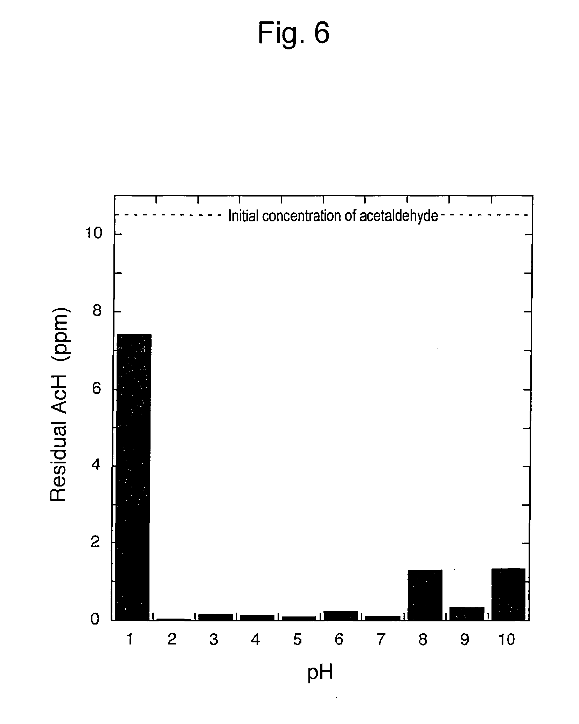

Fig. 6 shows the results of measuring acetaldehyde (AcH). Fig. 6 shows the concentrations of remaining acetaldehyde when acetaldehyde with the initial concentration of 10.5±0.5ppm was degraded by cells of the genus *Gluconobacter.* As shown in Fig. 6, the cells exhibited acetaldehyde-degrading capacity over a wide pH range between 2 and 10. In particular, sufficient degrading activity was observed in an acidic region. It was suggested that cells can exhibit excellent AcH-degrading capacity also within the stomach or intestine.

Based on the results of the above Examples, biomass required for removing acetaldehyde within the oral cavity after alcohol drinking is estimated as follows.

The acetaldehyde degradation rate per mg of freeze-dried *G*. *kondonii* NBRC3266 cells ranged from 4 to 5 µg • min⁻¹ • (mg dry cells)⁻¹. The concentration of acetaldehyde in saliva after alcohol drinking was reported to range from 2 to 4 ppm in many cases (Yokoyama A, Tsutsumi E, Imazeki H, Suwa Y, Nakamura C, Mizukami T, Yokoyama T. Alcohol Clin. Exp. Res. 32, 1607-1614 (2008)). For example, a standard amount of saliva (amount of saliva after stimulation) that is secreted after chewing gum for 10 minutes was reported to be 11 ml (Miwa et al., Journal of Japanese Society for Evidence and the Dental Professional 1, 40-43 (2009)), and this amount is regarded as the amount of salive present within the oral cavity. If 10 ppm acetaldehyde (110 µg) is present in 11 ml of saliva within the oral cavity after alcohol drinking, it is predicted that when 30 mg of freeze-dried cells are caused to exist within the oral cavity, acetaldehyde can be eliminated within 1 minute. Furthermore, based on the results of Example 5, if saliva containing 10 ppm acetaldehyde is secreted from the salivary gland at a rate of 1 ml per minute, it is predicted that the concentration of acetaldehyde within the oral cavity can be maintained at about 0.2 ppm or less when 60 mg of freeze-dried cells is present within the oral cavity during the period.

### [Example 8] Removal of odor substance

*G. kondonii* NBRC3266 was culutred by the method described in Example 1, the thus obtained cells were subjected to freeze-drying. 10 mg of freeze-dried cells was suspended in 500 µL of MilliQ water.

The cell suspension (50 µL) was added to 550 µL of 25 mM potassium phosphate buffer (pH7.0) containing 0.5 ppm nonenal or 0.5 ppm hexanal, followed by 5 minutes of reaction at 37°C. 60 µL of 6 M perchloric acid was added to the reaction solution to stop the reaction. Nonenal or hexanal contained in the reaction solution was measured by the same method as that described in comparative example 1.

As a result, cells efficiently degraded these aldehydes, so that nonenal or hexanal could not be detected in the reaction solution after the reaction had been stopped.

### [Example 9] Effects of ingestion of Gluconobacter kondonii cells on removal of acetaldehyde within the oral cavity after alcohol drinking

A subject (51 years old, body weight of 70 kg; ALDH2*1/*1) had breakfast (sliced bread, vegetables, milk) at 6 o'clock in the morning, and then fasted for 11 hours. The subject chewed chewing gum for saliva sampling before alcohol drinking, and then 2 ml of saliva was sampled.

Whisky (Yamazaki 10 years old; 40% alcohol) was diluted with mineral water, so that whisky with water (13% alcohol; acetaldehyde concentration of 18.2 ppm) was prepared. The subject ingested 90 ml of the whisky with water within 8 minutes. This was repeated 4 times, so that the subject ingested a total of 360 ml of whisky with water within 32 minutes (total alcohol ingestion: 47 g; 0.67 g/kg body weight). During ingestion, the subject ingested no supplementary food (snacks).

Immediately after ingestion of the total amount of alcohol, the subject chewed chewing gum for saliva sampling, and then 2 ml of saliva was sampled. The sampled saliva was immediately cooled with ice, and 500 µl of the sample was mixed with 50 µl of 6 M perchloric acid. For a subject group that had ingested no *Gluconobacter kondonii* cells (twice: control), 2 ml of saliva was sampled at 5 minutes, 30 minutes, 60 minutes, 90 minutes, 120 minutes, and 180 minutes after completion of alcohol drinking.

Meanwhile, in the case of a subject group that had ingested *Gluconobacter kondonii* cells (*G*. *kondonii* NBRC3266), the subject ingested *Gluconobacter kondonii* cells (30 mg of freeze-dried cells) immediately after the first saliva sampling, and the cells were dispersed uniformly within the oral cavity. In the 1st experiment, the subject chewed chewing gum for saliva sampling at 5 minutes after ingestion, and then 2 ml of saliva was sampled together with cells. In the 2nd experiment, after swallowing of cells at 5 minutes after ingestion, the subject chewed chewing gum for saliva sampling, and then 2 ml of saliva was sampled. At 30 minutes, 60 minutes, 90 minutes, 120 minutes, and 180 minutes after completion of alcohol drinking, 2 ml of saliva was sampled. In the 2nd experiment, saliva was also sampled at 15 minutes after completion of alcohol drinking.

The concentration of acetaldehyde in sampled saliva was measured by head-space gas chromatography (n = 2). In addition, all experiments were conducted for the same subject, and an interval of 2 days or more was provided between each experiment and the next experiment. The specific activity of the ingested *Gluconobacter kondonii* cells was 4 U/mg.

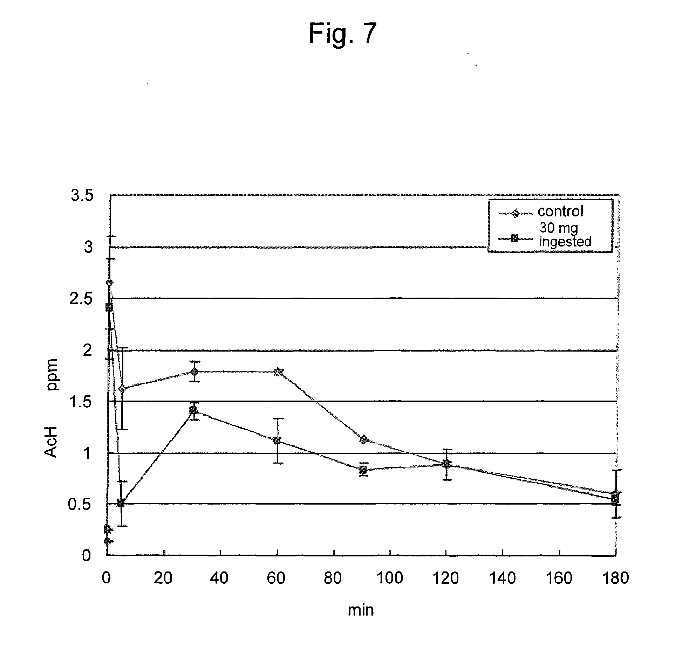

Fig. 7 shows the change in the concentration of acetaldehyde within the oral cavity after alcohol drinking. In Fig. 7, the horizontal axis indicates the time (min) after alcohol drinking. Immediately after alcohol drinking, a pulse-like increase of about 2.5 ppm was observed in the concentration of acetaldehyde. In the case of a subject who had ingesting no *Gluconobacter kondonii* cells ("control" in the graph in Fig. 7), the concentration of acetaldehyde within the oral cavity remained at 1.8 ppm for about 1 hour after alcohol drinking, and then decreased to 1.1 ppm after 90 minutes, 0.9 ppm after 120 minutes, and then 0.6 ppm after 180 minutes.

Meanwhile, in the case of a subject group that had ingested *Gluconobacter kondonii* cells ("30 mg ingested" in the graph in Fig. 7), the concentration of acetaldehyde within the oral cavity decreased to 0.5 ppm immediately after ingestion, increased again after expectoration or incorporation of the cells, and then reached 1.4 ppm at 30 minutes, 1.1 ppm at 1 hour, and then 0.8 ppm at 90 minutes after completion of alcohol drinking. Regarding the concentration of acetaldehyde within the oral cavity, there was no significant difference between the subject group that had ingested *Gluconobacter kondonii* cells and the subject group that had not ingested *Gluconobacter kondonii* cells at 120 minutes and 180 minutes after alcohol drinking. Within 90 minutes after alcohol drinking, the concentration of acetaldehyde within the oral cavity after ingestion of *Gluconobacter kondonii* cells was significantly lower than in the case in which no *Gluconobacter kondonii* cells had been ingested.

It was revealed for the first time by the experiment that once acetaldehyde within the oral cavity was removed and then cells were removed, the concentration of acetaldehyde started to increase again. This may be because: saliva containing acetaldehyde at an *in vivo* equilibrium concentration of 1 ppm-1.5 ppm is continuously secreted from the salivary gland; or acetaldehyde is continuously generated from ethanol remaining *in vivo.* However, it is noteworthy that within 90 minutes after alcohol drinking, the concentration of acetaldehyde within the oral cavity in the case in which *Gluconobacter kondonii* cells had been ingested was significantly lower than the case in which no *Gluconobacter kondonii* cells had been ingested. This may be because *Gluconobacter kondonii* cells remain due to being capatured by the fine structure within the oral cavity, for example, and degrade acetaldehyde.

It was suggested that the concentration of acetaldehyde within the oral cavity can be more effectively maintained at a low level if a method that allowed *Gluconobacter kondonii* cell colonization for a longer time period within the oral cavity through prescripton of a formulation could be devised.

### [Example 10] Effects by addition of Gluconobacter kondonii cells to foods

*G. kondonii* NBRC3266 was cultured by the method described in Example 1, and then the thus obtained cells were subjected to freeze-drying. The possibility of acetaldehyde (AcH) generation with combinations of the thus obtained cells and foods listed in Table 1 below was examined.

50 µl (dry wt, 1 mg) of a cell suspension (20 mg dry wt/mL) was added to a food (400 µl) pre-incubated to 37°C, followed by 5 minutes of reaction at 37°C. Then the resultant was mixed with 50 µl of 6 M perchloric acid.

Subsequently, changes in AcH content were measured by head-space gas chromatography. As a result, no significnat AcH generation was observed with mixture of foods listed in Table 1 and the cells.

**[Table 1]**

| | |
|---|---|
| Foods tested | |

| Category | Food (Manufacturer) |
|---|---|
| Milk | Brand 1 |
| | Brand 2 |
| Soymilk | Brand 1 |
| | Brand 2 |
| Fermented milk | Yogurt brand 1 |
| | Brand 2 |
| Pickled vegetable | Brand 1 (Eggplant) |
| | Brand 2 (Cucumber) |
| Cold beverage | Brand 1 (100% Orange) |
| | Brand 2 (100% Apple) |
| | Brand 3 (Tomato juice) |
| | Brand 4 (Cola) |
| Canned coffee | Brand 1 |
| | Brand 2 |
| Instant coffee | Brand 1 |
| Tea drink | Brand 1 (Jawa tea) |
| | Brand 2 (Green tea) |
| | Brand 3 (Barley tea) |
| | Brand 4 (Oolong tea) |
| Nutritious drink | Brand 1 |
| Consommé soup | Brand 1 |
| Instant miso soup | Brand 1 |
| Instant noodle | Cup noodle |
| Seasoning | Whole soybean soy sauce |
| | Semi-thick sauce |
| | Mellow true mirin (sweet cooking rice wine) |

All publications, patents, and patent applications cited herein are incorporated herein by reference in their entirety.

## Claims

1. An aldehyde-removing composition comprising a microorganism belonging to the genus *Gluconobacter* having aldehyde-degrading activity, or an extract thereof or disrupted cells thereof, wherein the microorganism belonging to the genus *Gluconobacter* has higher aldehyde-degrading activity than aldehyde-generating activity.

2. The aldehyde-removing composition according to claim 1, wherein the microorganism belonging to the genus *Gluconobacter* can degrade aldehyde under conditions where the alcohol concentration is higher than the aldehyde concentration.

3. The aldehyde-removing composition according to claim 1, wherein the microorganism belonging to the genus *Gluconobacter* is a microorganism belonging to *Gluconobacter kondonii* or *Gluconobacter kanchanaburiensis.*

4. The aldehyde-removing composition according to claim 3, wherein the microorganism belonging to *Gluconobacter kondonii* or *Gluconobacter kanchanaburiensis* is the microorganism specified with NBRC3266 or NBRC103587.

5. The aldehyde-removing composition according to claim 1, wherein the aldehyde is acetaldehyde.

6. The aldehyde-removing composition according to claim 2, wherein the alcohol is ethanol.

7. The aldehyde-removing composition according to claim 1, which is a composition that reduces the risk of developing upper gastrointestinal cancer.

8. The aldehyde-removing composition according to claim 7, wherein upper gastrointestinal cancer is selected from the group consisting of oral cancer, pharyngeal cancer, and esophageal cancer.

9. The aldehyde-removing composition according to claim 1, which is a composition for deodorization.

10. The aldehyde-removing composition according to claim 9, wherein the odor is a dead-wine smell.

11. The aldehyde-removing composition according to claim 1, which is used for removing acetaldehyde within the oral cavity after alcohol drinking.

12. A beverage or a food comprising the aldehyde-removing composition of any one of claims 1 to 11.
